# EUROPÄISCHE PATENTSCHRIFT

(11) **EP 0 951 481 B1**
(45) Veröffentlichungstag und Bekanntmachung des Hinweises auf die Patenterteilung: **24.11.2004**
(21) Anmeldenummer: 97951843.8
(22) Anmeldetag: 10.12.1997
(51) Int. Cl.: C07K 16/28, C07K 14/705

(54) **VERWENDUNG VON SUBSTANZEN MIT IMMUNMODULATORISCHER WIRKUNG FÜR DIE BEHANDLUNG DER AMYOTROPHEN LATERALSKLEROSE**
USE OF SUBSTANCES WITH IMMUNOMODULATING ACTIVITY FOR THE TREATMENT OF AMYOTROPHIC LATERAL SCLEROSIS
UTILISATION DE SUBSTANCE A EFFET IMMUNOMODULATEUR POUR LE TRAITEMENT DE LA SCLEROSE LATERAL AMYOTROPHIQUE

(43) Veröffentlichungstag der Anmeldung: 27.10.1999
(73) Patentinhaber: MPB MelTec Patent- und Beteiligungsgesellschaft mbH, 39120 Magdeburg (DE)
(72) Erfinder: Schubert, Walter, Dr., 39175 Biederitz (DE)
(74) Vertreter: Hofstetter, Alfons J., Dr.rer.nat.
(86) Internationale Anmeldenummer: PCT/DE1997/002883
(87) Internationale Veröffentlichungsnummer: WO 1999/029731

(56) Entgegenhaltungen:
- EP-A- 0 614 978
- WO-A-91/08301
- DE-A- 19 723 690
- SCHUBERT ET AL.: "Detection by 4-parameter microscopic imaging and increase of rare mononuclear blood leukocyte types expressing..." NEUROSCIENCE LETTERS, Bd. 198, Nr. 1, 22.September 1995, Seiten 29-32, XP002073560

## Beschreibung

Die vorliegende Erfindung betrifft die Verwendung von mindestens einer Verbindung zur Herstellung eines Arzneimittels zur Behandlung von amyotropher Lateralsklerose (ALS).

Die amyotrophe Lateralsklerose (im folgenden mit der Kurzform ALS bezeichnet) ist eine ätiologisch ungeklärte, in der Regel innerhalb von 3 bis 5 Jahren letal verlaufende, therapeutisch bisher nicht oder nicht signifikant beeinflussbare neurodegenerative Krankheit des Motoneuronsystems beim Menschen. Der progrediente Untergang von Nervenzellen des ersten und des zweiten motorischen Neurons sind die Ursache für eine zunehmende Lähmung der Willkürmuskulatur, bis hin zur völligen Gehunfähigkeit und der zunehmenden Lähmung der Atemmuskulatur. Es ist weitgehend gesichert, dass zelluläre und humorale (antikörpervermittelte) immunologische Prozesse in der Pathogenese der ALS eine wichtige, allerdings im einzelnen noch ungeklärte Rolle spielen. Die Krankheit tritt weltweit mit einer Prävalenz von 4 auf 100.000 und einer Inzidenz von 1 auf 100.000 Einwohner auf.

Zahlreiche Untersuchungsergebnisse sprechen dafür, dass immunologische Mechanismen bei der Pathogenese der amyotrophen Lateralsklerose eine Rolle spielen. Folgende Befunde stützen diese Annahme: Zytotoxische Serumaktivität von ALS Patienten in neuronalen Zellkulturen; Serum Immunglobulin G (IgG) Toxizität gegen spinale und cortikaie Neurone sowie gegen spannungsabhängige Kalzium Kanalproteine; Zytotoxizität der Cereobrospinalflüssigkeit von ALS-Patienten gegen Glutamat Rezeptoren; Veränderungen der Serum Konzentration von IgG Isotypen; Immunantwort peripherer Blutlymphozyten von ALS-Patienten gegen isolierte Zellmembranen; Nachweis invasiver Zellen des Immunsystems im Motoneuronsystem von ALS Patienten. Hier scheinen diese Zellen an den Mechanismen der Schädigung motorischer Nervenzellen beteiligt zu. sein (die Zitate dieser einzelnen Daten finden sich in: Westarp, M.E. und Mitarb., Neuosci. Lett. 173, 124-126, 1994).

Obwohl diese Daten eine Beteiligung des zellulären Immunsystems bei ALS wahrscheinlich machen, konnten eindeutige quantitative oder qualitative Veränderungen im zellulären Immunsystem bei der ALS bisher nicht nachgewiesen werden. Insbesondere wurden bisher im Blut bei Patienten mit ALS keine ALS-spezifischen Zellformen gefunden, die aufgrund ihrer molekularen Zelloberflächenmerkmale erkennbar sind und sich deutlich von Immunzellen gesunder Probanden oder anderen neurologischen Krankheiten unterscheiden. Dennoch wurden zahlreiche Therapieversuche mit relativ breit wirksamen und ungerichtet wirkenden Medikamenten zur Unterdrückung oder Modulation von Immunfunktionen unternommen. Diese Therapieversuche blieben ohne gesicherten Therapieerfolg (siehe z.B. Brown, R.H. und Mitarb.: Arch. Neurol. 43, 383-384, 1986; und z.B. Dalakas, M.C. und Mitarb.: Arch. Neurol. 51, 861-864, 1994). Nachteil dieses Vorgehens ist, dass diese Therapieformen (blind bzw. in Unkenntnis spezifischer Zellparameter bei der ALS) nicht zielgerichtet sind, d.h. nicht ausschließlich nur solche Zellformen blockieren oder unterdrücken, die bei der ALSspezifisch vorkommen oder signifikant erhöht vorkommen.

In der EP-A-0614978 werden lösliche menschliche Fcγ-Rezeptoren, Verfahren zu deren Herstellung, arzneiliche Zusammensetzungen die diese enthalten, deren Verwendung als Arzneimittel und deren diagnostische Verwendung offenbart. Die pharmazeutische Präparate enthalten dabei den löslichen FcγRIII-Rezeptor oder einen Antikörper, der an den FcγRIII-Rezeptor bindet. Die WO 91 08301 beschreibt ein Protein, welches die Interaktion zwischen dem Fc-Fragment eines Immunoglobulins und seines Rezeptors verhindert. Des weiteren wird eine therapeutische Verwendung, insbesondere für die Behandlung von Erkrankungen, die verwandt mit dem HIV-Virus sind, beschrieben. Dabei wird auf das Protein CD16 hingewiesen.

Es wurde in eigenen Untersuchungen gefunden, dass im Blut bei Patienten mit amyotropher Lateralsklerose mononukleäre Zellen des Immunsystems vorkommen, die bei keiner anderen neurologischen Krankheit oder Gesunden beobachtet werden (ALS-spezifische Immunzellen, siehe Tabelle 1). Im Gegensatz zu den bisher bei ALS beobachteten erhöht vorkommenden Immunzellformen, die als Stand der Technik bekannt sind (Schubert, W.: Neurosci Lett. 198, 29-32, 1995) erweisen sich die in Tabelle 1 erfindungsgemäß entdeckten Zellformen als vollkommen spezifisch für die ALS. Diese Zellformen exprimieren Rezeptoren für Immunglobuline (Fcγ-Rezeptoren), vorzugsweise für Immunglobulin G (IgG) der Subklasse 1 (IgG1) und 3 (IgG3) (im folgenden als FcγRIII bezeichnet). Die Vermehrung bzw. das ALS spezifische Auftreten dieser Immunzellen erklärt die bisher unverstandene Erniedrigung von IgG1 und IgG3 im Serum bei Patienten mit der Diagnose ALS, die z.B. von Westarp und Mitarbeitern berichtet wurde (Westarp, M.E. und Mitarb., Neuosci. Lett. 173, 124-126, 1994). FcγRIII-Rezeptoren binden diese Immunglobuline selektiv, d.h. eine Vermehrung von Zellen, die solche Rezeptoren exprimieren (wie in eigenen Untersuchungen entdeckt), führt zu einer verstärkten Bindung von IgG1 und IgG3 und damit zu einer IgG1-IgG3-Clearance bei dieser Krankheit. Es wurde in eigenen Untersuchungen erstmals gefunden, dass diese Fcγ-Rezeptorpositiven Zellen auf der Zelloberfläche eine Reihe von molekularen Merkmalen der Aktivierung aufweisen und in Zellkultur zu einer Schädigung bzw. Zerstörung von Nervenzellen führen. Diese Zellen stehen daher in einem direkteren Zusammenhang mit dem Pathomechanismus bei der ALS und stellen als Zellform erstmals eine konkrete Zielstruktur für eine gezielte, d.h. spezifische Therapie mit immunwirksamen Substanzen dar.

Aufgabe der vorliegenden Erfindung ist es daher, eine Verwendung von Substanzen für die Behandlung der ALS zur Verfügung zu stellen.

Die vorliegende Erfindung beruht auf der Entdeckung ALS-spezifischer bzw. bei ALS signifikant vermehrter, Fcγ-Rezeptor-positiver, speziell FcγRIII-positiver aktivierter Zellformen im Blut und besteht in einer Verwendung von Substanzen zur selektiven Unterdrückung, Zerstörung oder selektiven funktionellen Blockierung dieser Zellformen oder Blockierung bzw. funktionellen Inaktivierung von Fcγ-Rezeptoren durch Infusion oder Injektion definierter Substanzen gemäss Anspruch 1.

Fcγ Rezeptoren sind in der Immunologie seit langem bekannt. Es existieren drei verschiedene, aber verwandte menschliche Fcγ-Rezeptorklassen: FcγRI, FcγRII, und FcγRIII. Die Aminosäuresequenzen der Mitglieder dieser Rezeptorfamilie sowie die diese Rezeptoren codierenden Gene sind bekannt: FcγRI (Allen, J.M., Seed, B.: Science 243, 378-380, 1989), FcγRII (Ravetch, J.V., Kinet, J.-P.: Annu.Rev. Immunol. 9, 457-492, 1991), FcγRIII (Ravetch, J.V., Perussia, B.: J. Exp. Med. 170, 481-497, 1989). Die meisten Unterformen dieser drei Rezeptorklassen sind in der Zellmembran bestimmter Immunzellen verankert. Alle drei Klassen enthalten jedoch auch lösliche, d.h. nicht in der Zellmembran verankerte Rezeptorproteine, die von Immunzellen natürlicherweise frei gesetzt werden. Die Mechanismen sind jedoch verschieden. Lösliche FcγRI-Rezeptoren werden durch ein Stop Codon in der extrazellulären Domäne generiert. Lösliche FcγRII-Rezeptoren werden durch alternatives RNA-Spleißen generiert, und lösliche FcγRIII-Rezeptoren werden durch proteolytische Spaltung des membranverankerten Rezeptors generiert (siehe zusammenfassende Übersicht in: Jan, G.J. und Mitarb.: Immunology Today, 14, 215-221, 1993).

Die genannten Fcγ-Rezeptoren sind für eine Reihe wichtiger immunologischer Funktionen von besonderer Bedeutung. Alle Funktionen beruhen darauf, dass Fcγ-Rezeptoren Immunglobuline der Klasse G spezifisch binden. Die Bindung solcher Immunglobuline an Fcγ-Rezeptoren stimuliert bzw. triggert eine Reihe verschiedener zellulärer Aktivitäten: Phagozytose, Endozytose, die antikörperabhängige zellvermittelte Zytotoxizität, die Freisetzung entzündungsfordemder löslicher Faktoren (Mediatoren) sowie die Verstärkung von Mechanismen der Antigenpräsentation. Immunglobuline G binden an Fcγ-Rezeptoren vermittels ihrer Fc-Teile. Wenn sich z.B. Immunglobuline G auf diese Weise an Fcγ-Rezeptoren binden, die auf der Oberfläche von bestimmten Immunzellen verankert sind, so können diese Immunzellen nunmehr in hoch spezifischer Weise an antigen-beladene Zielzellen binden und diese zerstören. Diese Mechanismen spielen wahrscheinlich bei zahlreichen sogenannten Autoimmunkrankheiten eine wichtige pathogenetische Rolle. In einzelnen Fällen konnte gezeigt werden, dass durch eine Blockierung oder funktionelle Inaktivierung solcher Mechanismen die krankheitsspezifische Zerstörung von Zielzellen weitgehend verhindert werden kann. Ein Beispiel ist die beim Menschen vorkommende, seltene Form einer Autoimmunkrankheit, die sog. akute immunthrombozytopenische Purpura, bei der es aufgrund Fcγ-Rezeptor vermittelter zytotoxischer Aktivität des Immunsystems gegen Blutplättchen zu einem akuten Absinken der Blutplättchen (Thrombozyten) im Blut der betroffenen Patienten kommt. Hier konnte gezeigt werden, dass durch Infusion löslicher Fragmente von Fcγ-Rezeptoren eine deutliche Besserung der Symptome und der zellulären Krankheitszeichen erreicht werden kann (Debre und Mitarb.: Lancet, 342, 945-948, 1993). Bei der ALS ist jedoch bisher ein immunologischer Pathomechanismus vermittels der Aktion von Fcγ-Rezeptoren nicht bekannt. Auch wurde eine auf die gezielte Inaktivierung oder Blockierung solcher Mechanismen gerichtete Therapieform nicht entwickelt oder durchgeführt. Nunmehr haben eigene Untersuchungsergebnisse die Existenz entsprechend spezifischer Zellformen und solcher Mechanismen ergeben.

Im einzelnen wurde gefunden,
dass im Blut bei Patienten mit ALS mononukleäre Zellen vorkommen, die Fcγ-Rezeptoren auf der Zelloberfläche tragen und zusätzlich bzw. gleichzeitig eine unterschiedliche Anzahl verschiedener anderer Rezeptorproteine tragen, die eine ungewöhnliche Form der Zelloberflächenaktivierung zeigen. Die zusammen mit Fcγ-Rezeptoren, vor allem FcγRIII-Rezeptoren, variabel ko-exprimierten Oberflächenrezeptoren sind in Tabelle 1 im einzelnen bezeichnet. Die genannten Kurzbezeichnungen (CD) folgen der internationalen Nomenklatur (siehe Barcley, A.N. und Mitarb.: The leukocyte antigen facts book. Academic press. London 1993). Diese besondere, in Tabelle 1 gezeigte Kombination von Fcγ-Rezeptoren (z.B. CD16) mit anderen Rezeptorproteinen ist spezifisch für die ALS, da sie bei Gesunden oder anderen neurologischen Kontrollkrankheiten oder anderen immunologischen, nicht-neurologischen Krankheiten nicht gefunden werden konnten;
dass diese Zellen nach Isolierung aus dem Blut von ALS-Patienten in Zellkulturmedien angezüchtet werden können (hier zugrunde liegende Standardprotokolle siehe Lindl, T., Bauer, J. Zell- und Gewebekultur, G. Fischer Verlag, Stuttgart, 1987);
dass diese Zellen nach Anreicherung und in einer Ko-Kultivierung mit Nervenzellen und Serum von ALS-Patienten eine zytotoxische Aktivität entfalten, die zur Zerstörung der Nervenzellen führt;
dass diese zytotoxische Aktivität durch Zugabe verschiedener löslicher Faktoren blockiert werden kann:
a) durch monoklonale Antikörper gegen Fcγ-Rezeptoren in einer Konzentration zwischen 20 und 100 µMol,
b) durch lösliche Fcγ-Rezeptoren in einer Konzentration zwischen 10 und 60 µMol,
c) durch anti-sense messenger-Ribonukleinsäure Spezies (mRNA), die komplementär zu Fcγ-Rezeptor-spezifischen mRNA Sequenzen sind (z.B. 20 µMol eines 23-er antisense Oligonucleotids, abgeleitet von EMBL/Genbank FcγRIIT Sequenz X16863),
d) durch aus Gardnerella vaginalis isoliertes Protein V (EP: 0595997) in Konzentrationen zwischen 150 bis 170 µMol bei gleichzeitiger Anwesenheit von Serum von ALS-Patienten.

Die erfindungsgemäße Lösung besteht demnach in der die Verwendung von mindestens einer der oben genannten Substanzen zur Herstellung eines Arzneimittels zur Behandlung von amyotropher Lateralsklerose (ALS) und deren Verabreichung, um die mit der Fcγ-Rezeptorvermittelte zytotoxische Aktivität der ALS-spezifischen Fcγ-Rezeptor-positiven Immunzellen zu blockieren bzw. Fcγ-Rezeptoren zu blockieren oder zu inaktivieren, oder diese zu zerstören, um die pathologischen direkten oder indirekten Wirkungen dieser Zellen oder der Fcγ-Rezeptoren auf das motorische Nervensystem zu verhindern.

Eine Anwendung der genannten Substanzen erfolgt daher in jeweils therapeutischer Dosierung und nach Verträglichkeitsprüfung im Bolus (siehe Beispiele). Richtwerte liegen z.B. in folgenden Konzentrationen: Für FcγR spezifische Antikörper 10 bis 1000 mg/kg Körpergewicht, 1 bis 2 mal pro Tag i.v., für Fcγ-Rezeptor spezifische anti-sense RNA 10 bis 20 mg/kg, für lösliche Fcγ-Rezeptoren 10 bis 1000 mg/kg und für Protein V 10 bis 1000 mg/kg. Eine genaue therapeutische Dosierung kann jedoch gegebenenfalls nur im Einzelfall entschieden werden. Sie richtet sich unter anderem nach dem Ansprechen der Fcγ-Rezeptorpositiven Zellen auf die Verabreichung der Substanzen gemäss Anspruch 1 und nach der individuellen Verträglichkeit, die durch einmalige Bolusgaben (s. Beispiel 1) ermittelt werden kann. Dieses Ansprechen kann z.B. durch die Bestimmung der Anzahl solcher Zellformen im Blut ermittelt werden oder durch in-vitro-Zytotoxizitätsassays. Gegebenenfalls müssen Dosierung und Verteilung der Tagesdosis individuell modifiziert werden. Die erfindungsgemäße Lösung weist folgende Vorteile auf:
1. Im Gegensatz zu allen bisher durchgeführten, im wesentlichen unspezifisch und das gesamte Immunsystem erfassenden immunsuppressiven oder immunmodulierenden Therapieformen, stellt die hier dargelegte erfindungsgemäße Therapieform eine gezielte, d.h. auf ALS-spezifische Immunzellformen gerichtete Therapieform dar.
2. Aufgrund der erfindungsgemäßen Kenntnis der ALS-spezifischen Oberflächenmerkmale, d.h. kombinatorischen Rezeptormuster dieser Zellen (siehe Tabelle 1) kann vor jeder Therapie getestet werden, ob diese Zellen vorliegen, oder, nach deren Isolierung und in-vitro-Testung, festgestellt werden, ob sie zytotoxische, im besonderen neurotoxische Aktivität aufweisen.
3. Aufgrund der erfindungsgemäßen Kenntnis der Zelloberfläcbenmerkmale dieser Zellen kann jeweils unter einer laufenden Therapie entsprechend der Verwendung gemäß Anspruch 1 der Therapieerfolg nicht nur klinisch, sondern auch zellulär gemessen werden, indem durch Blutabnahrnen geprüft wird, ob die Fcγ-Rezeptor-positiven Zellformen infolge der Therapie numerisch gemindert werden oder gar aus der Blutzirkulation verschwinden.
4. Aufgrund der unter 3 erhobenen Befunde kann jeweils die Dosis der verabreichten Substanzen angepasst, d.h. erhöht oder erniedrigt werden, in Abhängigkeit davon, ob die Fcγ-Rezeptor-positiven Zellformen ansprechen oder nicht.
5. Da mit der erfindungsgemäßen Kenntnis der Zelloberflächenmerkmale der ALS-spezifischen Zellformen erstmals ein krankheitsspezifischer Zellparameter bei ALS besteht, kann der klinische Verlauf exakt mit diesem Parameter korreliert werden, sodass auch nach Absetzen der Therapie entsprechend der erfindungsgemäßen Verwendung Fcγ-Rezeptorspezifischer Substanzen ein möglicher Wiederänstieg der Fcγ Rezeptor-positiven Zellen eine genaue Bestimmung des Zeitpunktes für einen erneuten Therapiezyklus erlaubt.
   Die erfindungsgemäße Lösung wird einschließlich ihrer Funktionsweise nachstehend anhand eines Ausführungsbeispiels näher erläutert.

### Beispiel 1:

Patient: X1
Diagnose: ALS, bulbäre Form
Substanz: lösliche Fcγ-Rezeptor-Präparationen, 50 kDa, aus E. coli (Fusionsproteine aus E. Coli)
Applikationsweise: intravenös
Therapieschema: Verträglichkeitsprüfung mit 10 bis 1000 mg/kg im Bolus i.v., dann 150 mg/kg Körpergewicht täglich, über 5 Tage.

**Tabelle 1**

| | ALS | | | | | | | | | | | | | | | | | Kontrolle |
|---|---|---|---|---|---|---|---|---|---|---|---|---|---|---|---|---|---|---|
| CD | 1 | 2 | 3 | 4 | 5 | 6 | 7 | 8 | 9 | 10 | 11 | 12 | 13 | 14 | 15 | 16 | 17 | n |
| CD16 | + | + | + | + | + | + | + | + | + | + | + | + | + | + | + | + | + | + |
| CD2 | + | + | + | + | + | - | - | - | - | - | - | - | - | - | - | - | - | - |
| CD3 | + | + | + | + | + | + | + | + | + | + | + | + | + | + | + | + | + | + |
| CD4 | + | + | + | + | + | + | + | - | + | + | + | + | + | + | + | - | - | + |
| CD8 | - | + | - | - | - | - | - | - | - | - | - | - | - | - | - | - | - | - |
| CD56 | - | - | + | + | + | + | + | + | + | + | + | - | - | - | - | + | + | - |
| CD57 | + | - | + | - | - | - | - | - | - | - | - | - | - | - | - | - | - | - |
| CD26 | - | + | - | - | - | - | - | - | - | - | - | - | - | - | - | - | - | - |
| CD38 | + | - | + | + | + | + | + | + | + | + | + | + | + | + | + | + | + | + |
| CD71 | - | + | - | - | - | - | - | - | - | - | - | - | - | - | - | - | - | - |
| HLA-DR | + | - | + | + | + | + | + | + | + | + | - | + | + | - | - | - | - | - |
| HLA-DQ | - | - | - | - | - | - | - | - | - | - | - | - | - | - | - | - | - | - |
| CD11b | + | - | - | - | - | + | + | - | - | - | - | + | - | + | + | - | - | - |
| CD45Ra | - | +- | - | - | - | - | - | - | - | - | - | - | - | - | - | - | | - |
| CD7 | + | - | + | - | - | + | - | + | - | - | - | - | + | - | - | - | - | - |
| CD62L | + | - | + | + | - | + | + | + | + | - | - | + | + | + | - | - | + | + |
| CD36 | - | + | - | | - | - | - | - | - | - | - | - | - | - | - | - | - | - |
| CD19 | - | | - | | - | - | - | - | - | - | - | - | - | - | - | - | - | - |
| CD: "Cluster of differentiation antigens" auf der Zelloberfläche isolierter mononukleärer peripherer Blutleukozyten; 1 bis 17: verschiedene ALS-spezifische Zellformen, die CD 16 (FcγRIII) in verschiedenen Kombinationen mit anderen CD-Oberflächenantigenen gleichzeitig auf der Zelloberfläche exprimieren; n: FcγRIII - positive normale immunzellform bei einem gesunden Probanden | | | | | | | | | | | | | | | | | | |

## Patentansprüche

1. Die Verwendung von mindestens einer Verbindung zur Herstellung eines Arzneimittels zur Behandlung von amyotropher Lateralsklerose (ALS), wobei die mindestens eine Verbindung umfasst:
- mindestens einen Antikörper, der an mindestens einen Fcγ-Rezeptor bindet und einzelne Spezies dieser Rezeptorfamilie inaktiviert oder zur Zerstörung der Zellformen führt, die diese Rezeptoren tragen,
und/oder
- mindestens einen löslichen Fcγ-Rezeptor, der Immunglobuline bindet,
und/oder
- ein aus Gardnerella vaginalis gewonnenes Immunglobulin G bindendes Protein V,
und/oder
- mindestens ein anti-sense RNA Molekül, das spezifisch an eine mRNA Sequenz des Fcγ-Rezeptors bindet.

2. Verwendung nach Anspruch 1, wobei der mindestens eine Antikörper mindestens einen monoklonalen Antikörper umfaßt.

3. Verwendung nach einem der Ansprüche 1 oder 2, wobei der mindestens eine Antikörper mindestens einen Antikörper umfaßt, der an einen FcγRIII-Rezeptor bindet.

4. Verwendung nach einem der vorhergehenden Ansprüche, wobei der mindestens eine lösliche Fcγ-Rezeptor mindestens einen löslichen FcγRIII-Rezeptor umfaßt.

5. Verwendung nach einem der vorhergehenden Ansprüche, wobei der mindestens eine lösliche Fcγ-Rezeptor mindestens einen löslichen Fcγ-Rezeptor umfaßt, der Immunglobuline der Subklasse 1 (IgG1) und/oder 3 (IgG3) bindet.

6. Verwendung nach einem der vorhergehenden Ansprüche, wobei das mindestens eine anti-sense RNA Molekül mindestens ein anti-sense RNA Molekül umfaßt, das spezifisch an eine mRNA Sequenz des FcγRIII-Rezeptors bindet.

7. Verwendung nach einem der vorhergehenden Ansprüche, wobei der mindestens eine Antikörper mindestens einen Antikörper umfaßt, der an eine zytotoxische Substanz gekoppelt ist.

## Claims

1. Use of at least one compound for producing a drug for treating amyotrophic lateral sclerosis (ALS), the at least one compound including:
- at least one antibody binding to at least one Fcγ receptor and inactivating individual species of this receptor family or resulting in destruction of the cell forms carrying these receptors,
and/or
- at least one soluble Fcγ receptor binding immunoglobulin
and/or
an immunoglobulin G binding protein V extracted from Gardnerella vaginalis, and/or
- at least one antisense RNA molecule specifically binding to an mRNA sequence of the Fcγ receptor.

2. Use according to claim 1, wherein the at least one antibody includes at least one monoclonal antibody.

3. Use according to any one of claims 1 or 2, wherein the at least one antibody includes at least one antibody binding to an FcγRIII receptor.

4. Use according to any one of the preceding claims, wherein the at least one soluble Fcγ receptor includes at least one soluble FcγRIII receptor.

5. Use according to any one of the preceding claims, wherein the at least one soluble Fcγ receptor includes at least one soluble Fcγ receptor binding immunoglobulins of subclass 1 (IgG1) and/or 3 (IgG3).

6. Use according to any one of the preceding claims, wherein the at least one antisense RNA molecule includes at least one antisense RNA molecule specifically binding to an mRNA sequence of the Fcγ receptor.

7. Use according to any one of the preceding claims, wherein the at least one antibody includes at least one antibody coupled to a cytotoxic substance.

## Revendications

1. L'utilisation d'au moins un composé pour la fabrication d'un médicament prévu pour le traitement de la sclerose latérale amyotrophique (ALS), l'au moins un composé comprenant:
- au moins un anticorps se combinant chimiquement avec au moins un récepteur Fcγ et désactivant différentes espèces de ce groupe de récepteurs ou menant à la destruction des formes de cellules portant ces récepteurs,
et/ou
- au moins un récepteur Fcγ soluble liant des immunoglobulines,
et/ou
- une protéine V liant une immunoglobuline G récupérée de Gardnerella vaginalis,
et/ou
- au moins une molécule RNA antisens se fixant spécifiquement à une séquence mRNA du récepteur Fcγ.

2. Utilisation selon la revendication 1 dans laquelle l'au moins un anticorps comporte au moins un anticorps monoclonal.

3. Utilisation selon l'une des revendications 1 ou 2 dans laquelle l'au moins un anticorps comporte au moins un anticorps se fixant à un récepteur FcγRIII.

4. Utilisation selon l'une des revendications précédentes dans laquelle l'au moins un récepteur Fcγ soluble comporte au moins un récepteur FcγRIII soluble.

5. Utilisation selon l'une des revendications précédentes dans laquelle l'au moins un récepteur Fcγ soluble comporte au moins un récepteur Fcγ soluble liant des immunoglobulines de la sous-classe 1 (IgGI) et/ou 3 (IgG3).

6. Utilisation selon l'une des revendications précédentes dans laquelle l'au moins une molécule RNA antisens comporte au moins une molécule RNA antisens se fixant spécifiquement à une séquence mRNA du récepteur FcγRIII.

7. Utilisation selon l'une des revendications précédentes dans laquelle l'au moins un anticorps comporte au moins un anticorps couplé à une substance cytotoxique.
